# EUROPÄISCHE PATENTSCHRIFT

(11) **EP 0 416 371 B1**
(45) Veröffentlichungstag und Bekanntmachung des Hinweises auf die Patenterteilung: **08.11.1995**
(21) Anmeldenummer: 90116041.6
(22) Anmeldetag: 22.08.1990
(51) Int. Cl.: A61B 1/00, G02B 23/26, G02B 23/16

(54) **Endoskopoptik**
Optic for endoscope
Optique pour endoscope

(30) Priorität: 04.09.1989 DE 3929285
(43) Veröffentlichungstag der Anmeldung: 13.03.1991
(73) Patentinhaber: Richard Wolf GmbH, 75438 Knittlingen (DE)
(72) Erfinder: Lehmann, Helmut, D-7527 Kraichtal-Menzingen (DE); Karst, Siegfried, D-7531 Eisingen (DE)
(74) Vertreter: Wilcken, Thomas, Dipl.-Ing.

(56) Entgegenhaltungen:
- EP-A- 0 369 936
- DE-A- 1 566 045
- GB-A- 2 149 936
- US-A- 3 261 349
- US-A- 4 871 229

## Beschreibung

Die Erfindung betrifft eine Endoskopoptik, im wesentlichen bestehend aus einem die optischen Bauelemente aufnehmenden Innenrohr und einem dieses umgebenden Außenrohr, wobei in dem zwischen diesen beiden Rohren gebildeten Raum Lichtleitfasern angeordnet sind.

Um eine gleichmäßige Ausleuchtung des Bildfeldes und eine gute Bildqualität erzielen zu können, müssen die Lichtleitfasern in dem Außenrohr möglichst konzentrisch um das die optischen Linsen aufnehmende Innenrohr herum angeordnet sein. Dazu werden bei der Fertigung der Endoskopoptiken die Lichtleitfasern gleichmäßig um das Innenrohr verteilt und beispielsweise mit einem Faden an diesem befestigt. Dann wird das Innenrohr mit den um seinen Umfang verteilten Lichtleitfasern in das Außenrohr eingeschoben. Da für diesen Fertigungsgang keine speziellen Zentriervorrichtungen vorhanden sind, kann es leicht vorkommen, daß die Lichtleitfasern beim Einschieben in das Außenrohr so aus ihrer Lage am Umfang des Innenrohres verdrängt werden, daß das Innen- und Außenrohr exzentrisch zueinander zu liegen kommen und das Innenrohr streckenweise nicht parallel zur Mittelachse des Außenrohres verläuft. Durch die daraus resultierende ungleiche Verteilung der Lichtleiter am distalen Ende ergibt sich eine mangelhafte, d.h. ungleiche Ausleuchtung des Bildfeldes und, auch infolge der abschnittsweise nicht parallelen Anordnung des die optischen Linsen aufnehmenden Innenrohres in bezug auf das Außenrohr, eine ungenügende Bildqualität. Das Problem der Zentrierung ist vor allem bei der Fertigung von dünnen und langen Optiken mit 0°-Blickrichtung besonders groß.

Aus der DE-OS 23 61 873 ist eine Vorrichtung zur konzentrischen Anordnung von vielen Lichtleitfasern kleinen Durchmessers um eine einzelne Lichtleitfaser großen Durchmessers bekannt. Hier werden die Lichtleitfaser großen Durchmessers durch eine zentral angeordnete Hülse einer Führungsplatte aus Holz und die Lichtleitfasern kleinen Durchmessers durch viele in der Führungsplatte in einem oder mehreren konzentrischen Kreisen um die zentrale Führungshülse herum angeordneten Führungshülsen hindurchgeführt.Auf diese Weise lassen sich bei der Fertigung von Endoskopoptiken zwar die Lichtleitfasern exakt auf dem Innenrohr anordnen, aber die beim Einschieben in das Außenrohr bestehende Schwierigkeit der Zentrierung dieser Einheit in dem Außenrohr wird damit nicht beseitigt.

Es ist daher die Aufgabe der Erfindung, bei einem Endoskop der einleitend angeführten Art eine koaxiale oder exakt achsparallele Anordnung von Innenrohr und Außenrohr und eine gleichmäßige Verteilung der Lichtleitfasern in dem Raum zwischen diesen beiden Rohren zu ermöglichen.

Diese Aufgabe wird erfindungsgemäß dadurch gelöst, daß bei dem einleitend angeführten Endoskop der genannte Raum durch längsverlaufende Stege in Form von Drähten überbrückt und in die Lichtleitfasern aufnehmende Teilräume unterteilt ist.

Bevorzugte Ausgestaltungen der Erfindung sind in den Unteransprüchen angegeben. Die Vorteile der erfindungsgemäßen Lösung bestehen insbesondere darin, daß die exakte gegenseitige Ausrichtung von Innenrohr und Außenrohr sicher erreicht wird und zwischen den Stegen abgegrenzte Teilräume für die Lichtleitfasern entstehen, die ihre gleichmäßige Anordnung ermöglichen, was eine gleichmäßige Ausleuchtung des Bildfeldes und eine gute Bildqualität ermöglicht.

Um eine Verlagerung der Stege und Lichtleitfasern beim Einführen des Innenrohres in das Außenrohr zu vermeiden, können die Stege und die Lichtleitfasern auf dem Innenrohr mindestens im Bereich des distalen Endes oder alternativ dahinter durch Kleben oder durch Zusammenbinden mittels Faden fixiert werden.

Die Erfindung ist nachstehend anhand eines in der Zeichnung dargestellten Ausführungsbeispieles näher erläutert.
Es zeigen:
- Figur 1: eine erfindungsgemäß ausgestaltete Endoskopoptik mit einem vergrößert dargestellten Längs-Teilschnitt durch den Endoskopschaft,
- Figur 2: einen Querschnitt durch den Endoskopschaft längs der Schnittlinie II-II in Figur 1,
- Figur 3: eine Montage-Hilfsvorrichtung für die Endoskopoptik nach Figur 1,
- Figur 4: einen Querschnitt durch die Montage-Hilfsvorrichtung nach Figur 3,
- Figur 5: einen Längs-Teilschnitt durch den Endoskopschaft einer Seitenblickendoskopoptik,
- Figur 6: einen Querschnitt durch den Endoskopschaft längs der Schnittlinie VI-VI in Figur 5.

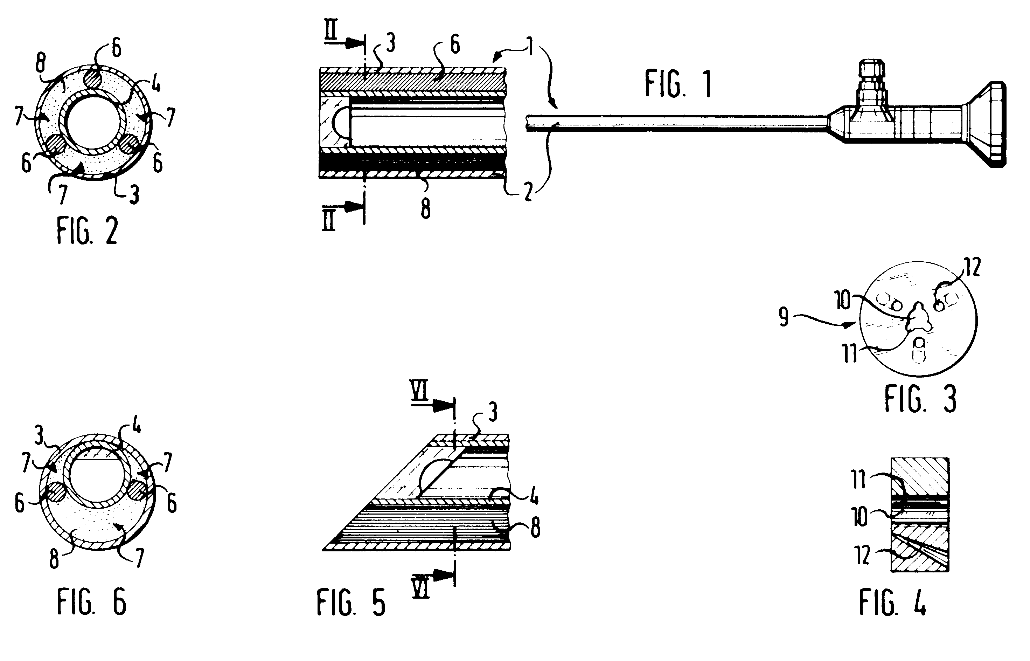

Die erfindungsgemäß ausgestaltete Endoskopoptik 1 mit 0°-Blickrichtung besteht gemäß Figuren 1 und 2 im wesentlichen aus einem den Endoskopschaft 2 bildenden Außenrohr 3 und einem die optischen Linsen aufnehmenden Innenrohr 4. Letzteres ist in dem Außenrohr 3 angeordnet und bildet einen zwischen diesem und dem Innenrohr befindlichen ringförmigen Raum. Für die zentrische Anordnung des Innenrohres 4 in dem Außenrohr 3 sind Stege 6 vorgesehen, im Ausführungsbeispiel drei, die den genannten Raum zwischen Innenrohr 4 und Außenrohr 3 überbrücken und zwischen sich Teilräume 7 bilden. Diese dienen der Aufnahme von Lichtleitfasern 8, die in einer Anzahl eingebracht werden, daß diese Teilräume 7 ausgefüllt sind.

Für das Einführen des Innenrohres mit den Lichtleitfasern 8 und den Stegen 6 in das Außenrohr 3 wird zweckmäßigerweise eine Montage-Hilfsvorrichtung 9 benutzt. Diese besteht aus einem scheibenförmigen Element, das zum Aufbau einer den Querschnitt nach Figur 2 aufweisenden Endoskopoptik eine Mittelbohrung 10 zur Aufnahme des Innenrohres 4 aufweist, die an ihrer Peripherie mit drei zu dieser achsparallelen Bohrungen 11 versehen ist. Weiter sind in einheitlichem radialem Abstand zur Mittelbohrung 10 vorzugsweise konische Bohrungen 12 angeordnet, die schräg zur Achse der Mittelbohrung 10 verlaufen.

Die Montage-Hilfsvorrichtung 9 wird zunächst mit ihrer Mittelbohrung 10 auf das Innenrohr 4 aufgesteckt. Durch die Bohrungen 11 hindurch werden dann die die Stege 6 bildenden Drähte zum distalen Ende des Innenrohres 4 geführt und dort z.B. durch Löten oder Kleben an dem Innenrohr 4 befestigt. Anschließend werden die Lichtleitfasern 8 jeweils durch die Bohrungen 12 geschoben, in die jeweiligen Teilräume 7 zwischen zwei benachbarten Stegen 6 eingelegt und dann zusammen mit den Stegen 6, z.B. mit einem Faden, wenigstens am distalen Bereich des Innenrohres 4 angebunden oder angeklebt. Beim Einschieben des Innenrohres 4 in das Außenrohr 3 gelangen, geführt durch die Bohrungen 11, 12 der Montage-Hilfseinrichtung 9, die Stege 6 und die Lichtleitfasern 8 in ihre auf dem Innenrohr 4 vorgesehene Lage.

Es ist auch möglich, die die Stege 6 bildenden Drähte und die Lichtleitfasern 8 in der gewünschter: Lage zu fixieren, indem sie hinter dem distalen Ende des Innenrohres 4 beispielsweise mit einem Faden festgebunden oder mittels eines Klebers festgelegt werden. Dadurch bleibt der Raum, den der Kleber oder das Lot zum Befestigen der Drähte auf dem Innenrohr 4 benötigen, frei, und es können mehr Lichtleitfasern 8 zwischen zwei Stegen 6 untergebracht werden. Die Anzahl der Stege 6 ist nicht auf die in der Zeichnung beispielhaft angegebene Anzahl beschränkt. Insbesondere erweist sich bei der Fertigung von Optiken mit von 0° abweichenden Blickrichtungen eine Anordnung nach den Figuren 5 und 6 mit einem oder zwei Stegen 6 mit koaxialem oder nicht koaxialem bzw. achsparallelen Innenrohr 4 sowie einer entsprechend modifizierten Montage-Hilfsvorrichtung als vorteilhaft.

Die drahtförmigen Stege 6 können aus einem Metall mit guten Gleiteigenschaften, wie z.B. Monel, gefertigt sein. Dabei sind prinzipiell alle geeigneten Drahtquerschnittsformen verwendbar. Besonders vorteilhaft ist die Verwendung von Stegen 6 aus lichtleitendem Material, da dann kein Lichtverlust durch die Stege gegeben ist.

## Patentansprüche

1. Endoskopoptik (1), bestehend aus einem optische Bauelemente aufnehmenden Innenrohr (4) und einem dieses umgebenden Außenrohr (3), wobei zwischen diesen beiden Rohren ein Raum gebildet ist, in dem Lichtleitfasern (8) angeordnet sind, dadurch gekennzeichnet, daß der genannte Raum durch längsverlaufende Stege (6) in Form von Drähten überbrückt und in die Lichtleitfasern (8) aufnehmende Teilräume (7) unterteilt ist.

2. Endoskopoptik nach Anspruch 1, dadurch gekennzeichnet, daß die Stege (6) aus lichtleitendem Material bestehen.

3. Endoskopoptik nach Anspruch 1 oder 2, dadurch gekennzeichnet, daß die Stege (6) und die Lichtleitfasern (8) auf dem Innenrohr (4) mindestens im Bereich des distalen Endes durch Kleben fixiert sind.

4. Endoskopoptik nach Anspruch 1 oder 2, dadurch gekennzeichnet, daß die Stege (6) und die Lichtleitfasern (8) auf dem Innenrohr (4) mitndestens im Bereich des distalen Endes durch Zusammenbinden mittels Faden fixiert sind.

5. Endoskopoptik nach Anspruch 1 oder 2, dadurch gekennzeichnet, daß die Stege (6) und die Lichtleitfasern (8) hinter dem distalen Ende des Innenrohres (4) durch Zusammenbinden mittels Faden fixiert sind.

6. Endoskopoptik nach Anspruch 1 oder 2, dadurch gekennzeichnet, daß die Stege (6) und die Lichtleitfasern (8) hinter dem distalen Ende des Innenrohres (4) durch Kleben fixiert sind.

## Claims

1. An endoscope optical system (1), consisting of an inner tube (4) holding optical components, and of an outer tube (3) surrounding it, wherein between these two tubes a space is formed in which photoconducting fibres (8) are arranged, characterised in that the said space is bridged by cross-pieces (6), running longitudinally, in the form of wires, and is divided into subspaces (7) holding the photoconducting fibres (8).

2. An endoscope optical system according to Claim 1, characterised in that the cross-pieces (6) consist of photoconducting material.

3. An endoscope optical system according to Claim 1 or 2, characterised in that the cross-pieces (6) and the photoconducting fibres (8) are fixed on the inner tube (4) at least in the region of the distal end by glueing.

4. An endoscope optical system according to Claim 1 or 2, characterised in that the cross-pieces (6) and the photoconducting fibres (8) are fixed on the inner tube (4) at least in the region of the distal end by binding together by means of thread.

5. An endoscope optical system according to Claim 1 or 2, characterised in that the cross-pieces (6) and the photoconducting fibres (8) are fixed behind the distal end of the inner tube (4) by binding together by means of thread.

6. An endoscope optical system according to Claim 1 or 2, characterised in that the cross-pieces (6) and the photocoducting fibres (8) are fixed behind the distal end of the inner tube (4) by glueing.

## Revendications

1. Optique d'endoscope (1), constituée par un tube intérieur (4) accueillant des composants optiques, et un tube extérieur (3) entourant le précédent, ces deux tubes formant entre eux un espace, dans lequel sont agencées des fibres optiques (8), caractérisée en ce que ledit espace est ponté par des entretoises longitudinales (6) sous la forme de fils métalliques et est subdivisé en des espaces partiels (7) accueillant les fibres optiques (8).

2. Optique d'endoscope selon la revendication 1, caractérisée en ce que les entretoises (6) sont réalisées en un matériau réalisant un guidage de la lumière.

3. Optique d'endoscope selon la revendication 1 ou 2, caractérisée en ce que les entretoises (6) et les fibres optiques (8) sont fixées par collage sur le tube intérieur (4), au moins dans la zone de l'extrémité distale.

4. Optique d'endoscope selon la revendication 1 ou 2, caractérisée en ce que les entretoises (6) et les fibres optiques (8) sont fixées sur le tube intérieur (4) par liaison ensemble au moyen de fils, au moins dans la zone de l'extrémité distale.

5. Optique d'endoscope selon la revendication 1 ou 2, caractérisée en ce que les entretoises (6) et les fibres optiques (8) sont fixées par liaison ensemble au moyen de fils, en arrière de l'extrémité distale du tube intérieur (4).

6. Optique d'endoscope selon la revendication 1 ou 2, caractérisée en ce que les entretoises (6) et les fibres optiques (8) sont fixées par collage, en arrière de l'extrémité distale du tube intérieur (4).
